# EUROPÄISCHE PATENTSCHRIFT

(11) **EP 0 690 036 B1**
(45) Veröffentlichungstag und Bekanntmachung des Hinweises auf die Patenterteilung: **22.10.1997**
(21) Anmeldenummer: 95110159.1
(22) Anmeldetag: 29.06.1995
(51) Int. Cl.: C07C 11/24, C07C 2/78, C01B 3/36

(54) **Verfahren zur Herstellung von Acetylen und Synthesegas**
Process for the preparation of acetylene aus synthesis gas
Procédé pour la préparation d'acétylène et de gaz de synthèse

(30) Priorität: 29.06.1994 DE 4422815
(43) Veröffentlichungstag der Anmeldung: 03.01.1996
(73) Patentinhaber: BASF AKTIENGESELLSCHAFT, 67056 Ludwigshafen (DE)
(72) Erfinder: Pässler, Peter, Dr., D-67069 Ludwigshafen (DE); Feser, Rainer, Dr., D-67269 Grünstadt (DE); Thelen, Hans-Günter, Dr., D-69221 Dossenheim (DE)
(74) Vertreter: Isenbruck, Günter, Dr.

(56) Entgegenhaltungen:
- DE-B- 1 126 858
- FR-A- 2 226 891

## Beschreibung

Die Erfindung betrifft Verfahren zur Herstellung von Acetylen und Synthesegas durch partielle Oxidation von Kohlenwasserstoffen mit Sauerstoff, wobei die Ausgangsgase zunächst getrennt vorgeheizt, in einer Mischzone intensiv vermischt, nach Durchströmen eines Brennerblocks zu Reaktion gebracht und anschließend schnell abgekühlt werden, wobei der Brennerblock eine Anzahl von durchgehenden Kanälen aufweist.

Die Herstellung von Acetylen und Synthesegas durch partielle Oxidation von Kohlenwasserstoffen ist in der Vergangenheit vielfach beschrieben worden (siehe u.a. DE 12 59 875, DE 29 47 005, US 2,765,359, US 3,242,224 und H. Pichler, "Acetylene from hydrocarbon-oxygen mixtures", "Chemical Engineering Progress, Vol. 61, No. 8). So hat insbesondere die partielle Oxidation von Methan (Erdgas) zu Acetylen und Synthesegas weltweit großtechnische Bedeutung erlangt.

Während es sich bei der alleinigen Herstellung von Synthesegas aus Erdgas durch partielle Oxidation um einen technisch einfachen Prozeß handelt, ist der mit der Acetylenherstellung gekoppelte Synthesegasprozeß an präzise räumliche, zeitliche und mengenmäßige Verhältnisse gebunden. So werden die Ausgangsstoffe Erdgas und Sauerstoff üblicherweise möglichst auf bis zu 700°C getrennt vorgeheizt, in einer Mischzone intensiv vermischt und nach Durchströmen eines Brennerblock zur Reaktion gebracht. Das Volumenverhältnis von eingesetztem Sauerstoff zu eingesetztem Erdgas beträgt dabei etwa 0,6.

Der Brennerblock besteht aus einer bestimmten Anzahl von Kanälen, in denen die Geschwindigkeit der reaktionsfähigen Sauerstoff/Erdgas-Mischung höher als die Flammengeschwindigkeit ist, um ein Durchschlagen der Flamme in den Mischraum zu verhindern. Der sich dem Brennerblock anschließende Reaktionsraum ist so bemessen, daß bei einer bestimmten Einsatzstoffmenge die Verweilzeit des acetylenhaltigen Reaktionsgases, des sog. Spaltgases, nur wenige Millisekunden beträgt. Nach dieser Zeit, innerhalb der sich die dem Temperaturniveau von 1500 bis 2000°C entsprechenden Gleichgewichte nicht einstellen können, werden die Reaktionsprodukte möglichst augenblicklich auf unter 300°C mit Wasser oder vorzugsweise Rückstandsöl abgeschreckt, damit das gebildete Acetylen nicht in Ruß und Wasserstoff zerfällt. Normalerweise arbeiten diese Prozesse bei Normaldruck oder leicht erhöhtem Druck. Neben Erdgas können alle gasförmigen bzw. leicht verdampfbaren Kohlenwasserstoffe unter etwas geänderten Verfahrensbedingungen zum Einsatz gelangen. Alle Verfahrensvarianten haben jedoch den Nachteil, daß eine einmal berechnete bzw. empirisch ermittelte Reaktorgröße die Kapazität für Acetylen und Rohsynthesegas in bestimmten Grenzen festlegt, wobei das Verhältnis von Acetylen zu Synthesegas annähernd gleich bleibt. Dies hat zur Folge, daß bei einer Verringerung des Acetylenbedarfs auch die Produktion des Koppelproduktes Synthesegas zurückgeht und damit die der im Produktionsverbund stehenden Anlagen, wie z.B. die Methanolproduktion. Aus der DE 1 126 858 ist zur Vermeidung von Instabilitäten der Flamme hinter dem Gasverteilerblock bei großem Querschnitt der in diesem angeordneten Kanäle vorgesehen, den Querschnitt dieser Kanäle auf einem Teil der Gesamtlänge zu verengen. Hierfür sind Einbauten in jedes einzelne Rohr erforderlich. Dies ist konstruktiv aufwendig und eine Auswechslung ist nur schwer möglich.

Der Erfindung liegt die Aufgabe zugrunde, die bekannten Verfahren zur Herstellung von Acetylen und Synthesegas durch partielle Oxidation von Kohlenwasserstoffen in der Weise zu verbessern, daß bei der Koppelproduktion von Acetylen und Synthesegas das Mengenverhältnis zwischen dem gewonnenen Acetylen und dem Synthesegas in weiten Grenzen variiert werden kann.

Diese Aufgabe wird dadurch gelöst, daß die Kanäle des Brennerblocks auf der Anströmseite von ebenen mit Perforationen versehenen ebenen Platten abgedeckt sind.

Das erfindungsgemäße Verfahren ermöglicht es, das Sauerstoff zu Kohlenwasserstoff-Verhältnis in weiten Grenzen zu variieren, ohne daß eine Vorzündung auftritt. Während bei bekannten Verfahren das Sauerstoff-zu Kohlenwasserstoff-Verhältnis nur in engen Grenzen variiert werden kann, ermöglicht das erfindungsgemäße Verfahren überraschenderweise eine so starke Erhöhung des Verhältnisses, daß der Gehalt an Acetylen im Spaltgas stark zurückgeht. Es ist offensichtlich, daß das erfindungsgemäße Verfahren, das mit Hilfe einer Abwandlung eines vorhandenen bekannten Brennerblocks durchgeführt werden kann, ohne jegliche Komplikation von einer hohen zu einer niedrigen Acetylenproduktion umgestellt werden kann. Das heißt, es ist von einem niedrigen zu einem hohen Einsatzstoffverhältnis umstellbar, was dem Brenner eine außerordentliche Anpassungsfähigkeit verleiht. Dies ist in der Praxis von größter Bedeutung.

Mit Gasgeschwindigkeiten von 50 bis 150 m/s, vorzugsweise 100 m/s in den rohrfömigen Kanälen des Brennerblocks (Blockrohren) und von 100 bis 300, vorzugsweise 200 m/s in der perforierten Platte, entsprechend einem Flächenverhältnis von 2 zu 1, hat ein mit einer bestimmten Anzahl von Kanälen versehener Brennerblock und damit Brenner (Reaktor) eine hohe Kapazitätsbreite und damit Anpassungsfähigkeit an den Produktebedarf.

Der Durchmesser und die Anzahl der Kanäle bestimmt die Nenn-Kapazität eines Brennerblocks bzw. Brenners. Technisch interessant sind erfindungsgemäß Durchmesser der Kanäle von 15 bis 45 mm, vorzugsweise von 20 bis 35 mm. Deren Anzahl liegt zwischen 50 bis 150, vorzugsweise bei 127. Die Bohrungen der perforierten Abdeckplatte liegen bei einem Durchmesser von 2,5 bis 7 mm, vorzugsweise bei 3 bis 6 mm.

Weitere Einzelheiten und Vorteile der Erfindung können dem anhand der Zeichnung erläuterten Ausführungsbeispiel entnommen werden. Es zeigen:
- Fig. 1 eine schematische Darstellung einer bekannten Apparatur zur Durchführung einer partiellen Oxidation;
- Fig. 2 eine vergrößerte Darstellung eines Schnitts durch den Brennerblock der Fig. 1;
- Fig. 3 eine stark vergrößerte Darstellung eines Ausschnitts aus einem Brennerblock zur Durchführung des erfindungsgemäßen Verfahrens;
- Fig. 4 eine Draufsicht auf eine Abdeckplatte gemäß der Erfindung.

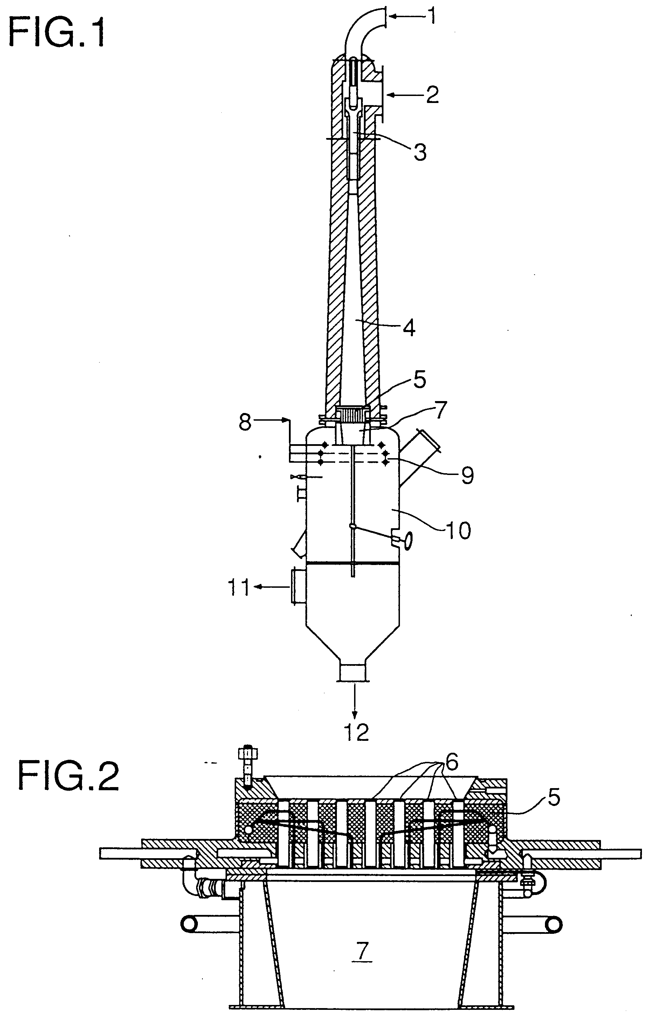

Die in den Figuren 1 und 2 dargestellte bekannte Apparatur ist dem Handbuch "Ullmann's Encyclopedia of Industrial Chemistry, Vol A1, 1985", Seite 107 entnommen. Dieser Apparatur werden durch die Leitung 1 Sauerstoff und durch die Leitung 2 Kohlenwasserstoff in vorerhitzten Zustand zugeführt. Die Komponenten werden in der Zone 3 so schnell vermischt, daß keine Bereiche mit so hoher Sauerstoffkonzentration entstehen, daß eine Vorzündung erfolgen könnte. Das Gemisch wird dann durch einen Diffusor 4 zum Brennerblock 5 und durch dessen Kanäle 6 in die Reaktionszone 7 geführt. Dort wird das Gemisch mit Hilfe einer Vielzahl von Hilfsflammen, die aus der Brennerblockunterseite, d.h. am Beginn des Reaktionsraumes austreten, augenblicklich und einheitlich gezündet.

Durch die Leitung 8 wird ein Kühlmittel über die Quenchrohre 9 dem Inneren des Quenchsbehälters 10 zugeführt. Durch die Leitung 11 verläßt das Spaltgas die Apparatur. 12 ist der Auslaß für das zur Quenchung verwendete Kühlmittel.

Bei dem in Fig. 2 dargestellten Brennerblock 5 sollen keine Details erläutert werden. Wesentlich erscheint hier im Hinblick auf die Erfindung nur, daß parallel verlaufende aus Bohrungen bestehende Kanäle 6 vorgesehen sind, die den Diffusor 4 mit dem Reaktionsraum 7 verbinden.

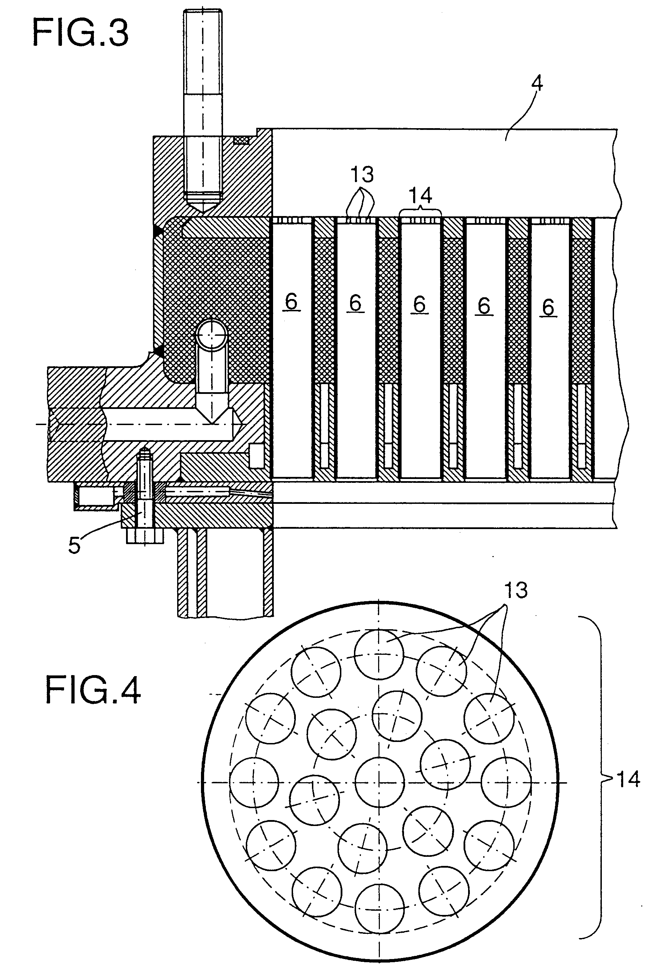

Fig. 3 zeigt einen vergrößerten und vereinfachten Schnitt durch einen der Fig. 2 entsprechenden Brennerblock 5. Der erfindungswesentliche Unterschied zum bekannten Brennerblock ist darin zu sehen, daß die Kanäle 6 am oberen, dem Reaktionsraum 7 entgegengesetzten Ende mit einem Plättchen 14 abgedeckt sind und dieses mit regelmäßig über seinen Querschnitt verteilten Bohrungen 13 versehen ist. Die Durchmesser der Kanäle 6 liegen in der Regel zwischen 15 mm und 45 mm, der Durchmesser der Perforationen liegt zwischen 2,5 mm und 7 mm, vorzugsweise bei etwa 4 mm. Die Dicke der Plättchen 14 kann zwischen 2 mm und 8 mm liegen, vorzugsweise bei etwa 3 mm.

Das Material der Plättchen muß hitzebeständig sein.

Anstatt die Kanäle des Brennerblocks einzeln mit perforierten Plättchen abzudecken, kann bei bestehenden Apparaturen auch eine den gesamten Brennerblock abdeckende Platte vorgesehen werden, die nur an den Stellen an denen die Kanäle des Brennerblocks münden mit entsprechenden Bohrungen versehen ist. In besonders einfacher Weise kann auch die gesamte Platte so mit Bohrungen versehen werden, daß jeweils über dem Ende der Kanäle die erfindungsgemäß erforderliche Anzahl von Bohrungen vorhanden ist.

In den folgenden Beispielen kam ein Brennerblock aus Stahl zum Einsatz, dessen Kanäle mit einem Durchmesser von 27 mm von je einem perforierten Plättchen derart abgedeckt wurden, daß 19 Bohrungen von je 4,2 mm Durchmesser auf jedes Blockrohr entfielen.

Die Dicke der Platte betrug 3 mm, das Material war Incoloy 800.

### Beispiel 1 bis 8:

Allen in nachfolgender Tabelle aufgeführten Beispielen ist gemeinsam, daß die Einsatzstoffe Erdgas und Sauerstoff getrennt auf 600 Grad Celsius aufgeheizt, in einer Mischeinrichtung 3 innig vermischt und nach Durchlaufen eines Diffusers 4 und des Brennerblocks 5 zur Reaktion gebracht wurden. Nach einer Reaktionszeit von wenigen Millisekunden wurden die acetylenhaltigen Spaltgase mit einem Schweraromatenöl auf ca. 250°C abgeschreckt und anschließend in bekannter Weise durch fraktionierte Absorption und folgende Desorption mit Hilfe eines geeigneten Lösemittels in die Produkte Acetylen und Rohsynthesegas zerlegt.

| Versuch | 1 | 2 | 3 | 4 | 5 | 6 | 7 | 8 |
|---|---|---|---|---|---|---|---|---|
| Nm3/h Sauerstoff | 4267 | 4255 | 4268 | 4264 | 3669 | 3674 | 3662 | 3668 |
| Nm3/h Erdgas | 6699 | 6207 | 5709 | 5216 | 5714 | 5294 | 4970 | 4544 |
| Verhältnis Sauerstoff zu Erdgas | 0,637 | 0,686 | 0,748 | 0,817 | 0,642 | 0,694 | 0,737 | 0,807 |
| Vol. % Acetylen im Spaltgas | 7,45 | 4,56 | 1,54 | 1,45 | 6,91 | 4,15 | 1,81 | 1,43 |
| Kg/h Acetylen | 1134 | 701 | 239 | 233 | 903 | 557 | 236 | 181 |
| Nm3/h Synthesegas | 11990 | 12500 | 13011 | 13468 | 10359 | 10965 | 10917 | 10629 |
| Nm3 Synthesegas pro Tonne Acetylen | 10573 | 17832 | 54439 | 57802 | 11472 | 19686 | 46258 | 58724 |

## Patentansprüche

1. Verfahren zur Herstellung von Acetylen und Synthesegas durch partielle Oxidation von Kohlenwasserstoffen mit Sauerstoff, wobei die Ausgangsgase zunächst getrennt vorgeheizt, in einer Mischzone intensiv vermischt, nach Durchströmen eines Brennerblocks zur Reaktion gebracht und anschließend schnell abgekühlt werden, wobei der Brennerblock eine Anzahl von durchgehenden Kanälen aufweist, **dadurch gekennzeichnet,** daß die Kanäle (6) des Brennerblocks (5) auf der Anströmseite von mit Perforationen (13) versehenen ebenen Platte (14) abgedeckt sind.

2. Verfahren nach Anspruch 1, dadurch gekennzeichnet, daß bei einem Durchmesser der Kanäle (6) von 15 mm bis 45 mm, vorzugsweise von 20 bis 35 mm, die Durchmesser der Perforationen (13) zwischen 2,5 mm und 7 mm, vorzugsweise bei 3 bis 6 mm liegen.

3. Verfahren nach Anspruch 1 und 2, dadurch gekennzeichnet, daß jeder einzelne Kanal (6) auf der Anströmseite eine separate Platte (14) mit Perforationen (13) aufweist.

4. Vorrichtung zur Herstellung von Acetylen und Synthesegas durch Oxidation von Kohlenwasserstoffen mit Sauerstoff mit einer Mischkammer (4), einem daran anschließenden Brennerblock (5) der mit Kanälen (6) versehen ist, die zu einem Reaktionsraum (7) führen, dadurch gekennzeichnet, daß die Kanäle (6) des Brennerblocks (5) auf der Anströmseite von mit Perforationen (13) versehenen ebenen Platten abgedeckt sind.

5. Vorrichtung nach Anspruch 4, dadurch gekennzeichnet, daß bei einem Durchmesser der Kanäle (6) von 15 mm bis 45 mm, vorzugsweise von 20 bis 35 mm, die Durchmesser der Perforationen (13) zwischen 2,5 mm und 7 mm, vorzugsweise bei 3 bis 6 mm liegen.

6. Vorrichtung nach Anspruch 4 oder 5, dadurch gekennzeichnet, daß jeder einzelne Kanal (6) auf der Anströmseite eine separate Platte (14) mit Perforationen (13) aufweist.

## Claims

1. A process for preparing acetylene and synthesis gas by partial oxidation of hydrocarbons with oxygen, the feedstock gases being first preheated separately, intensively mixed in a mixing zone, reacted after flowing through a burner block and then rapidly cooled, the burner block having a number of continuous ducts, wherein the ducts (6) of the burner block (5) are covered on the inlet side by a plane plate (14) furnished with perforations (13).

2. A process as claimed in claim 1, wherein with a duct diameter (6) of from 15 mm to 45 mm, preferably of from 20 to 35 mm, the diameters of the perforations (13) are from 2.5 mm to 7 mm, preferably from about 3 to 6 mm.

3. A process as claimed in claim 1 or 2, wherein each individual duct (6) has on the inlet side a separate plate (14) having perforations (13).

4. An apparatus for preparing acetylene and synthesis gas by oxidation of hydrocarbons with oxygen having a mixing chamber (4), a burner block (5) connected thereto which is furnished with ducts (6) which lead to a reaction compartment (7), wherein the ducts (6) of the burner block (5) are covered on the inlet side by a plane plate furnished with perforations (13).

5. An apparatus as claimed in claim 4, wherein with a duct diameter (6) of from 15 mm to 45 mm, preferably of from 20 to 35 mm, the diameters of the perforations (13) are from 2.5 mm to 7 mm, preferably from about 3 to 6 mm.

6. An apparatus as claimed in claim 4 or 5, wherein each individual duct (6) has on the inlet side a separate plate (14) having perforations (13).

## Revendications

1. Procédé de préparation d'acétylène et de gaz de synthèse par oxydation partielle d'hydrocarbures avec de l'oxygène, dans lequel les gaz de départ sont tout d'abord préchauffés de façon séparée, mélangés dans une zone de mélange de façon énergique, mis en réaction après avoir traversé un bloc brûleur puis finalement refroidis rapidement, le bloc brûleur présentant une série de canaux débouchants, caractérisé en ce que les canaux (6) du bloc brûleur (5) sont recouverts du côté du flux entrant, par une plaque plane (14) munie de perforations (13).

2. Procédé selon la revendication 1, caractérisé en ce que les diamètres des perforations (13) se situent entre 2,5 mm et 7 mm, de préférence à environ 3-6 mm, pour un diamètre des canaux (6) de 15 mm à 45 mm, de préférence de 20 à 35 mm.

3. Procédé selon la revendication 1 et 2, caractérisé en ce que chaque canal (6) présente du côté du flux entrant, une plaque séparée (14) munie de perforations (13).

4. Dispositif pour la préparation d'acétylène et de gaz de synthèse par oxydation d'hydrocarbures avec de l'oxygène, comportant une chambre de mélange (4), suivie par un bloc brûleur (5) muni de canaux (6), qui mènent à une chambre de réaction (7), caractérisé en ce que les canaux (6) du bloc brûleur (5) sont recouverts du côté du flux entrant, par une plaque plane munie de perforations (13).

5. Dispositif selon la revendication 4, caractérisé en ce que les diamètres des perforations (13) se situent entre 2,5 mm et 7 mm, de préférence à environ 3-6 mm, pour un diamètre des canaux (6) de 15 mm à 45 mm, de préférence de 20 à 35 mm.

6. Dispositif selon la revendication 4 ou 5, caractérisé en ce que chaque canal (6) présente du côté du flux entrant, une plaque séparée (14) munie de perforations (13).
